# EUROPEAN PATENT SPECIFICATION

(11) **EP 1 113 023 B1**
(45) Date of publication and mention of the grant of the patent: **09.11.2005**
(21) Application number: 99912076.9
(22) Date of filing: 31.03.1999
(51) Int. Cl.: C08B 37/00, C08B 15/00, A61K 39/39, A61K 9/00, C08L 1/00

(54) **METHOD OF FORMING AGGLOMERATES OF POLYSACCHARIDE WITH HYDROPHOBIC GROUPS**
VERFAHREN ZUR HERSTELLUNG VON AGGLOMERATEN AUS POLYSACCHARIDEN MIT HYDROPHOBEN GRUPPEN
PROCEDE DE FORMATION D'AGGLOMERATS DE POLYSACCHARIDE AVEC DES GROUPES HYDROPHOBES

(43) Date of publication of application: 04.07.2001
(73) Proprietor: NOF CORPORATION, Tokyo 150-6019 (JP)
(72) Inventor: HOSOTANI, Ryuzo, Tsukuba-shi Ibaraki 305-0045 (JP); HAYASHI, Akio, Chiba 277-0831 (JP); NAKANO, Yoshio, Tsukuba-shi Ibaraki 305-0045 (JP)
(74) Representative: Bannerman, David Gardner
(86) International application number: PCT/JP1999/001684
(87) International publication number: WO 2000/059948

(56) References cited:
- WO-A-98/09650
- JP-A- 3 292 301
- JP-A- 61 069 801
- JP-A- 63 319 046

## Description

The present invention relates to a process for forming aggregates (associated products) of hydrophobic group-containing polysaccharide.

Hydrophobic group-containing high molecular weight polysaccharides in which hydrophobic group(s) are bound to polysaccharide are used for medicinal materials, for example, coating material for coating a drug carrier enclosing therein a drug. It is known that, by coating a drug carrier, for example, liposome microcapsule, microsphere, O/W emulsion or erythrocyte ghost, with a hydrophobic group-containing polysaccharide, not only the spontaneous exudation of drug from such a drug carrier is suppressed but also the cell-specific drug transference rate using such a drug carrier is improved.

It has in recent years been widely accepted that liposome and O/W emulsion are prospective as drug carrier. It has been reported that the chemical and physical stabilities of a drug carrier of this kind within and without living body are improved by coating the drug carrier with polysaccharide, wherein thereby a target-tropism to a specific cell group is also revealed {Bull. Chem. Soc. Japan, 62, 791 - 796 (1989)}. It has further been reported that liposomes are physically stabilized by coating them with polysaccharide {Drug Delivery System, 5, 261 (1990)}.

Further, it is reported that hydrophobic group-containing polysaccharides interact with proteins and with compounds exhibiting higher hydrophobicity so as to encapsulate these proteins or compounds {Chem. Lett., 1263 (1991)}. In this literature is described that, when aggregates of a hydrophobic group-containing polysaccharide are mixed with a globular protein of varying kind at room temperature, the protein becomes coupled with the aggregates of the hydrophobic group-containing polysaccharide to form a conjugate. Therein is also described that aggregates of hydrophobic group-containing polysaccharides are stable even in the presence of excess amounts of such proteins.

Further, a vaccine product containing a hydrophobic group-containing polysaccharide and an antigen is also known (WO 98/09650). It is furthermore known that a conjugate of a hydrophobic group-containing polysaccharide and an antigen can be isolated and purified by mixing aggregates of the hydrophobic group-containing polysaccharide with the antigen at room temperature and, then, treating the resulting mixture by gel chromatography (Macromolecules, 27 7654-7659 (1994)).

On the other hand, Akiyoshi et al disclose in Macromolecules, 26 3062-3068 (1993) that a hydrophobicized polymeric substances are subject to intra- or intermolecular self association of their hydrophobic groups in a dilute aqueous solution, resulting in formation of aggregates of the polymer molecules. In particular, hydrophobic group-containing polysaccharide forms relatively monodisperse microparticles of aggregates in a size of nano-order in a dilute aqueous solution by spontaneous association of several molecules. It is confirmed by observation under electron microscope that relatively monodisperse globular microparticles of nano-order size are formed. Such relatively monodisperse nano-order size aggregates of hydrophobic group-containing polysaccharide exist in water as a dispersion which is colorless and transparent in appearance and does not form any cloud or precipitate even after standing still for a long period of time, leaving an appearance of aqueous solution in human eye.

By causing a hydrophobic group-containing polysaccharide to swell in water and agitating the resulting swollen dispersion using, for example, a homomixer, a turbid dispersion is obtained. In such a turbid dispersion, a part of the hydrophobic group-containing polysaccharide forms aggregates of a size of nano-order, while there are at the same time some which are present as lumps of various sizes without forming such aggregates. When a turbid liquid, in which lumps of sizes greater than nano-order size are present, is used for a medicinal material, for example, as a material in a drug delivery system (DDS) for intravenous administration, thrombus may be formed due to the above-mentioned lumps. When, on the other hand, the colorless transparent liquid in which the hydrophobic group-containing polysaccharide is present, forming aggregates of uniform nano-order size, is used therefor, there is no fear of thrombus formation. Therefore, there is a demand for aggregates of hydrophobic group-containing polysaccharide which are dissolved (dispersed in a colorless transparent state) in water, in order to use the hydrophobic group-containing polysaccharide as, for example, a medicinal material for building up a conjugate with a varying kind of drug or protein.

In the past, processes have been known for forming hydrophobic group-containing polysaccharide into aggregates, for example, 1) a process in which the hydrophobic group-containing polysaccharide is dissolved in dimethyl sulfoxide (DMSO) under a dilute condition and the resulting solution is then dialyzed against water and 2) a process in which the hydrophobic group-containing polysaccharide is caused to swell in water and the resulting swollen dispersion is then treated by ultrasonication (Macromolecules, 26 3062-3068 (1993); WO 98/09650).

It is, however, quite difficult to prepare such aggregates of hydrophobic group-containing polysaccharide industrially in large scale by the above-mentioned processes of prior art. Firstly, for example, in the dialysis process of the prior art, there are problems in that 1) a dialysis arrangement capable of large scale treatment is required, 2) a huge amount of water is necessary and 3) a prolonged period of time is required for the treatment. Secondly, in the process by ultrasonication of the prior art, there are problems in that 1) the throughput of one single treatment is lower, 2) deviation between treating lots is large, since control of sonication efficiency and of sonication time is difficult and monodisperse aggregates are not able to obtain steadily and 3) probable contamination with, for example, fractured metal fragments occurred due to deterioration of the sonication tip may occur.

On the background described as above, a large scale preparation of aggregates of hydrophobic group-containing polysaccharide is difficult by the processes with dialysis and ultrasonication of the prior art. Therefore, a more simple and convenient process for forming aggregates of hydrophobic group-containing polysaccharide is expected. However, no technique of forming aggregates of hydrophobic group-containing polysaccharide has hitherto been known other than the above-mentioned processes of dialysis and ultrasonication.

While a homogenizer is used for emulsifying oils in water, no practical experience has heretofore been known in which a homogenizer is used for forming aggregates of hydrophobic group-containing polysaccharide.

The object of the present invention is to obviate the problems in the prior art described above and to provide a process for forming aggregates of hydrophobic group-containing polysaccharide, in which the deviation between treating lots and the contamination by impurities are eliminated and which can afford to prepare uniform aggregates of hydrophobic group-containing polysaccharide steadily in a simple and convenient way within a brief time in large scale.

The inventors reached from their sound researches a knowledge that aggregates of a hydrophobic group-containing polysaccharide can be obtained in a simple and convenient way within a brief time in large scale by dispersing a swollen liquor of the hydrophobic group-containing polysaccharide using a high pressure homogenizer, whereby the present invention has been completed. Thus, the present invention consists in the process for forming aggregates of hydrophobic group-containing polysaccharide as set out in the claims.
Fig. 1 shows the results of Example 1-1 in graphs, each in a chart of the results of SEC analyses of a pullulan-cholesterol derivative (CHP) before and after the treatment by a high pressure homogenizer. Figs. 1(a) and 1(b) are each a chart of analysis result of SEC before and after the treatment of the CHP by the high pressure homogenizer, respectively. The ordinate represents the strength (dimensionless) of the differential refractometer (the same applies to those in the following).
Fig. 2 shows the results of Examples 1-2 to 1-5 in graphs, wherein Figs. 2(a), 2(b), 2(c) and 2(d) are each a chart of analytical result of SEC after the treatment by high pressure homogenizer for Examples 1-2, 1-3, 1-4 and 1-5, respectively.
Fig. 3 shows the result of Comparative Example 1 in a graph, a chart of the result of SEC analysis after the dialysis.
Fig. 4 shows by charts of results of SEC analyses of pullulan (of a molecular weight of 108,000) and of CHP. Figs. 4(a), 4(b) and 4(c) are each a chart of the result of SEC analysis, for the pullulan (molecular weight 108,000), for the CHP and for the aggregates of the CHP, respectively.
Fig. 5 is a chart of the result of SEC analysis of CHP (a concentration of 0.2 % by weight) after an ultrasonication for a predetermined period of time.
Fig. 6 shows the results of Comparative Example 2 in graphs, namely, charts of the SEC analysis results of a CHP after an ultrasonication treatment and after a treatment by a high-pressure homogenizer, respectively. Figs. 6(a) is a chart of the result of SEC analysis of the CHP after the ultrasonication treatment. Fig. 6(b) is a chart of the result of SEC analysis of the ultrasonicated liquor of Fig. 6(a) after it is treated by the high-pressure homogenizer.

The polysaccharides of the invention have -XH groups (wherein X denotes oxygen atom or a nitrogen-containing group represented by NY with Y standing for hydrogen atom or a hydrocarbon group of 1 - 10 carbon atoms), wherein 0.1 - 10, preferably 0.1 - 6, -XH groups per 100 monosaccharide units constituting the polysaccharide are replaced by one or more hydrophobic groups represented by the formula (1) given in claim 1.

As the hydrocarbon group having 1 - 50 carbon atoms represented by R¹ in the above formula (1), there may be enumerated, for example, radicals of ethylene, butylene, hexamethylene and diphenylmethane.

As the hydrocarbon group having 12 - 50 carbon atoms or the steryl group represented by R² in the above formula (1), there may be enumerated, for example, laulyl, myristyl, cetyl, stearyl, cholesteryl, stigmasteryl, β-sitosteryl, lanosteryl and ergosteryl.

For the polysaccharide to be substituted by hydrophobic groups (in the following, denoted sometimes as the pre-substitution polysaccharide) represented by the formula (1) given above for the hydrophobic group-containing polysaccharide, those of natural occurrence and semisynthetic ones are used. The pre-substitution polysaccharide may be selected from the group consisting of pullulan, amylopectin, amylose, dextran, hydroxyethyl cellulose, hydroxyethyl dextran, mannan, levan, inulin, chitin, chitosan, xyloglucan and water-soluble cellulose. Among them, pullulan, mannan, xyloglucan, amylopectin, amylose, dextran and hydroxyethyl cellulose are preferred. Polysaccharides having nitrogen atom(s), such as chitin, partially deacetylated chitin and chitosan, are also favorable. The polysaccharides may be employed either alone or in a combination of two or more of them.

The hydrophobic group-containing polysaccharide having the hydrophobic groups represented by the above formula (1) can be produced by a known technique. For example, it can be produced by a method, in which a diisocyanate compound represented by the formula OCN-R¹-NCO (in which R¹ denotes a hydrocarbon group having 1 - 50 carbon atoms) is reacted with a hydroxyl group-containing hydrocarbon having 12 - 50 carbon atoms or a sterol represented by the formula R²-OH (in which R² denotes a hydrocarbon group having 12 - 50 carbon atoms or a steryl group) in the first reaction step in which one mole of the hydroxyl group-containing hydrocarbon having 12 - 50 carbon atoms or the sterol is reacted to form an isocyanato group-containing hydrophobic compound, whereupon, in the second reaction step, the isocyanato group-containing hydrophobic compound obtained in the first reaction step is reacted with the pre-substitution polysaccharide mentioned above.

Concrete examples of the diisocyanate compound (OCN-R¹-NCO) to be used in the first reaction step include ethylene diisocyanate, butylene diisocyanate, hexamethylene diisocyanate and diphenylmethane diisocyanate, namely, those in which R¹ is a radical of ethylene, butylene, hexamethylene and diphenylmethane, respectively.

As the hydroxyl group-containing hydrocarbon (R²-OH) having 12 - 50 carbon atoms to be used in the first reaction step, there may be enumerated, for example, those originated from alcohols, such as lauryl alcohol, myristyl alcohol, cetyl alcohol, stearyl alcohol, arachidic alcohol, docosanol, pentacosanol, hexacosanol and octacosanol. Among them, those having 12 - 35 carbon atoms, in particular, those having 12 - 20 carbon atoms are preferred because of their easy availability. As the sterol (R²-OH), there may be enumerated, for example, cholesterol, stigmasterol, β-sitosterol, lanosterol and ergosterol.

An example of the second step reaction is shown below by the reaction schemes (I) and (II). In the reaction schemes given below, pullulan is employed as the pre-substitution polysaccharide. In the reaction scheme (I), a diisocyanate compound represented by the formula (2) is reacted with a sterol represented by the formula (3) to form the stearyl isocyanate represented by the formula (4). In this reaction, usually sterol dimer represented by the formula (5) is formed as a by-product. In the reaction scheme (II), the stearyl isocyanate represented by the formula (4) obtained by the reaction scheme (I) is reacted with pullulan represented by the formula (6) to produce a polysaccharide-sterol derivative (hydrophobic group-containing polysaccharide) represented by the formula (7).

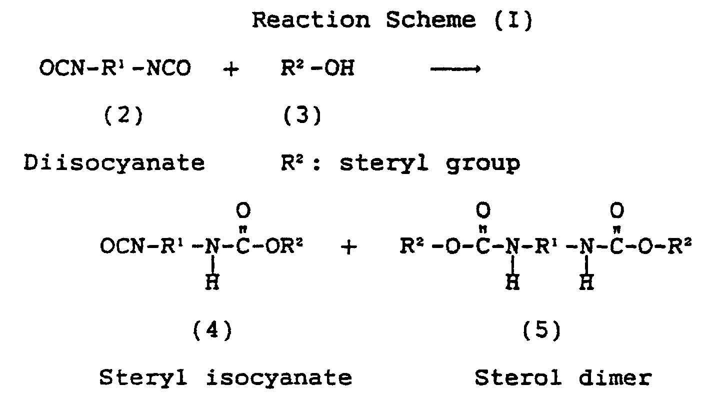

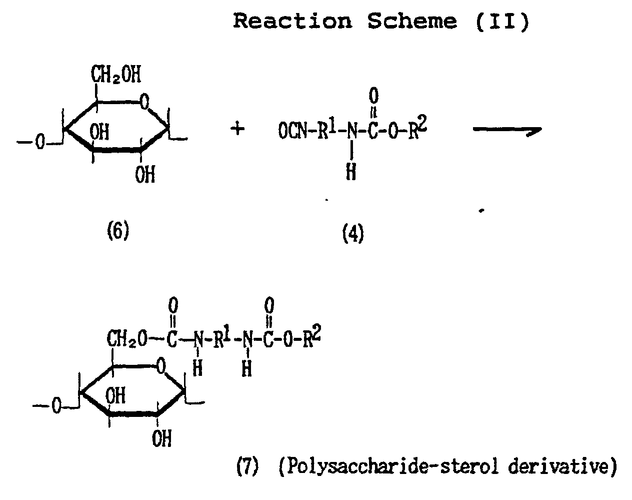

The hydrophobic group-containing polysaccharide obtained by the above reactions can be purified by means of a known technique, such as precipitation or centrifugation. By the purification, the sterol dimer (by-product) represented by the formula (5) can be removed. The hydrophobic group-containing polysaccharide having hydrophobic groups represented by the formula (1) and the production process thereof are given in detail in, for example, Japanese Patent Kokai Hei 3-292301 A and in Macromolecules,. 3062 (1993).

The process for forming the aggregates according to the present invention can be realized by the process steps [1] and [2] given in the following.

### Process step [1]

The hydrophobic group-containing polysaccharide is caused to swell in water.

### Process step [2]

The swollen dispersion obtained in the above process step [1] is subjected to a dispersing treatment using a homogenizer under a pressure of 9.8 - 490 MPa (100 - 5,000 kgf/cm²). The dispersing treatment of the process step [2] may be effected in two or more repeats. By repeating several times, the state of dispersion of the aggregates becomes more stable.

Below, the process for forming according to the present invention will further be described in more detail.

The amount of water to be used in the process step [1] may favorably be 30 - 10,000 times weight, preferably 100 - 1,000 times weight of the hydrophobic group-containing polysaccharide. If this amount is short of 30 times weight, the hydrophobic group-containing polysaccharide may become unfavorable gelled state. If this amount exceeds over 10,000 times weight, the efficiency of forming aggregates will become unfavorably decreased. While there is no special restriction as to the water temperature for effecting swelling, a temperature of 0 - 100 °C, preferably 10 - 50 °C, may be favorable.

The resulting swollen dispersion may favorably be brought to the subsequent process step [2] after having been stirred by a stirrer. As the stirrer to be employed, a magnetic stirrer, a homomixer or the like may be exemplified. Among them, preference is given to homomixer. While there is no special limitation for the revolution rate, stirring duration and so on of the stirrer, a revolution rate of 100 - 15,000 rpm and a stirring duration of 30 seconds to 180 minutes may be favorable. The dispersion resulting from stirring of the swollen dispersion is present as a turbid liquid, which gives birth to deposition of precipitate after standing for a while.

The homogenizer to be employed in the process step [2] should be capable of dispersion-treating the swollen dispersion from the process step [1] under a pressure of 9.8 - 490 MPa (100 - 5,000 kgf/cm²), preferably 98 - 294 MPa (1,000 - 3,000 kgf/cm²). For such a homogenizer, commercial high pressure homogenizer may be employed. A high pressure homogenizer is a device for attaining emulsification or microdispersion of a liquor by generating shearing forces, impingement momentums and cavitation by the aid of a high pressure.

When such a high pressure homogenizer is used, aggregates of the hydrophobic group-containing polysaccharide can be formed, concretely, in the following manner. First, the swollen dispersion is pressurized at a pressure mentioned above and the so-pressurized swollen dispersion is spouted from an orifice into a chamber to cause cavitation (pressure drop). The spouted swollen dispersion is thereby accelerated and is caused to bring about intense collisions of domains of the swollen dispersion with each other in the chamber and with the walls of the chamber. By the thereby generated impingement momentums and shearing forces, the hydrophobic group-containing polysaccharide is dispersed finely in the dispersion to build up aggregates thereof. The so-obtained treated liquor is present as a transparent colorless liquid which is a dispersion (expressed in the following sometimes as aqueous solution) not subject to occurrence of turbidity or precipitation after a prolonged standing still.

The dispersing treatment using high pressure homogenizer may be effected only once or in two or more repeats. The treatment with high pressure homogenizer may be carried out in a batchwise or continuous operation. While the number of repeats of the high pressure homogenizer treatment may vary considerably depending on, for example, each specific hydrophobic group-containing polysaccharide, the degree of substitution with such hydrophobic group, the concentration in the aqueous dispersion and the pressure on the high pressure homogenizer treatment, a stable and relatively monodisperse aggregate may be obtained usually by five repeats, though not affirmable. For example, in the case where the hydrophobic group-containing polysaccharide is a pullulan-cholesterol derivative with a cholesterol-substitution degree of 1.2 cholesterol groups per 100 monosaccharide units, the concentration in the aqueous dispersion is 0.2 % by weight and the pressure on the high pressure homogenizer treatment is 98 MPa (1,000 kgf/cm²), a stable aggregate without suffering from occurrence of turbidity or precipitation can be obtained by repeating the dispersing treatment by the high pressure homogenizer three times.

Concrete examples of the high pressure homogenizer which can be used in the process according to the present invention include MICROFLUIDIZER (of the firm Microfluidex, trademark), MICROFLUIDIZER (of Mizuho Kogyo K.K., trademark), DeBEE 2000 (trademark, supplied from Q.P. Corp.) and APV GAULIN (trademark, of APV Rannie, Inc.).

While there is no special limitation as to the temperature of the swollen dispersion on the dispersing treatment by a homogenizer, a temperature in the range from 0 to 100 °C, preferably from 10 to 50 °C, may be favorable.

By performing the dispersing treatment using a homogenizer, a monodisperse aggregate can be formed. The resulting monodisperse aggregate, namely, the aggregate of the hydrophobic group-containing polysaccharide obtained by the process according to the present invention, has usually an aggregate particle size in the range from 10 to 30 nm and a number of associations of the hydrophobic group-containing polysaccharide in the aggregate in the range from 3 to 20. Here, the particle size and the number of associations refer both to the average value. The resulting treated dispersion is a colorless transparent aqueous solution which will not become turbid nor bring about precipitation after a prolonged standing still. Here, the monodisperse aggregate will not be formed by simply agitating the swollen dispersion by a stirrer, such as a magnetic stirrer or homomixer. The swollen dispersion keeps its turbid state and will not turn into colorless transparent state even though the revolution rate of the stirrer is increased or the stirring is continued for prolonged period of time.

The aggregate of the hydrophobic group-containing polysaccharide formed by the process according to the present invention can be separated in a form of a solid matter by drying the aggregate, after it has been formed, by means of, for example, freeze-drying. From this solid matter, a colorless transparent aqueous solution of the aggregate in the state before the freeze-drying can be restored by adding water to the solid matter.

The aggregate of the hydrophobic group-containing polysaccharide formed by the process according to the present invention can be used as a medicinal material, such as a coating material for coating a drug carrier enclosing therein a drug. Thus, it can be used as a coating material for coating a drug carrier made of, for example, liposome, microcapsule, microsphere, O/W emulsion or erythrocyte ghost. Here, the aggregate of the hydrophobic group-containing polysaccharide obtained by the process according to the present invention can be used securely as a medicinal material and for preparing a drug carrier of stable quality, since the so-obtained aggregate is a homogeneous product and has no quality deviation between production lots nor contamination by impurity. The aggregate of hydrophobic group-containing polysaccharide obtained- by the process according to the present invention can also be utilized as surfactants, thickening agents and a raw material for cosmetics.

In the process according to the present invention, it is possible to employ a mixture of hydrophobic group-containing polysaccharide with one or more polysaccharides having no hydrophobic group (i.e. those before introduction of hydrophobic group therein) and/or one or more medicaments and/or one or more proteins, instead of using the hydrophobic group-containing polysaccharide solely. Hereby, the possibility of extension of application field, for example, in the drug delivery system (DDS), may be prospective.

As described above, aggregates of hydrophobic group-containing polysaccharide can securely be formed in a homogeneous quality steadily within a brief period of time, in a large scale and in a simple manner, by the process according to the present invention without suffering from quality deviation between production lots and from contamination by impurity, since the process is performed by a dispersing treatment of a swollen dispersion of the hydrophobic group-containing polysaccharide using a homogenizer under a pressure within a specific range.

Below, the present invention will concretely be described by way of Examples, though these Examples should not be regarded as restricting the scope of the present invention.

In all the Examples, the experimental conditions employed were as follows:

### « Conditions of Size Exclusion Chromatography (SEC) »

1) Apparatus used: TOSOH HPSEC SYSTEM (trademark, of Tosoh Ltd.)
2) Column: TSK-gel G4000SWXL (trademark, of Tosoh Ltd.)
3) Eluent: 0.05 % NaN₃ in deionized water
4) Flow rate: 0.5 ml/min.
5) Temperature: 35°C
6) Detector: RI (a differential refractometer)

### « Determination of Particle Size by Dynamic Light-Scattering Measurements »

Apparatus used: DLC-700 (trademark, of Otsuka Electronics Co., Ltd.)
Conditions of Determination: 5 mW He-Ne laser (633 nm); temperature = 25 °C; scattering angle = 25°; concentration = 4.15 mg/ml

### « Determination of Number of Assocoations by Static Light-Scattering Measurements »

Apparatus used: DLC-700 (trademark, of Otsuka Electronics Co., Ltd.)
Conditions of Determination: MR-102 (differential refractometer); temperature = 25°C; scattering angle = 30° - 130°; concentration = 0.72 - 1.93 mg/ml

### Synthesis Example 1-1

### « Synthesis of N-(6-isocyanatohexyl)cholesteryl carbamate »

An eggplant type 1-liter flask was charged with 25 grams (0.065 mol) of cholesterol and thereto were added 300 ml of toluene to dissolve it, whereto 17 ml (0.12 mol) of triethylamine were added. To this, 161 grams (0.96 mole, 14.8 eq.) of hexamethylene diisocyanate dissolved in 300 ml of toluene were added to cause a reaction at 80 °C for 6 hours under a nitrogen atmosphere. After termination of the reaction, toluene and the excess amount of hexamethylene diisocyanate were removed by reducing the pressure. The resulting yellowish oily residue was stood still overnight at room temperature to cause precipitation of pale yellow crystals. The crystals were taken out and about one liter of hexane was added thereto, whereupon the mixture was shaken vigorously and, then, the supernatant liquid was removed by decantation. This washing procedure was repeated four times, whereupon the crystals were dried under a reduced pressure at room temperature for three hours, whereby N-(6-isocyanatohexyl)cholesteryl carbamate represented by the following formula (4a) was obtained.

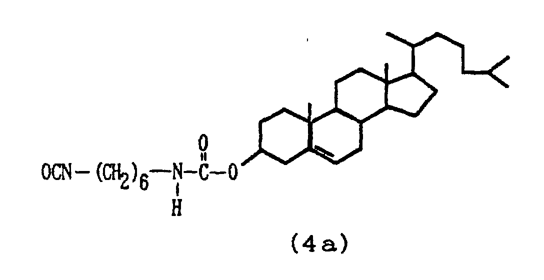

### Synthesis Example 1-2

### « Synthesis of N-(6-isocyanatohexyl)stearyl carbamate »

In an eggplant type flask of 300 ml capacity, there were charged 3.48 g (12.9 mmol) of stearyl alcohol and thereto were added 50 ml of toluene to dissolve it, whereto 2.04 g (25.8 mmol) of pyridine were further added. To this mixture, there were added 30 g (178 mmol, 14.8 eq.) of hexamethylene diisocyanate dissolved in 50 ml of toluene and the resulting mixture was subjected to reaction at 80 °C under a nitrogen atmosphere for about 3 hours. After termination of the reaction, toluene and the excess of hexamethylene diisocyanate were removed under a reduced pressure, whereby a pale yellow crystals were formed. The crystals were taken out, whereto about one liter of hexane was added and the mixture was shaken vigorously, whereupon the supernatant was removed by decantation. This washing procedure was repeated four- times, whereupon the product was dried under a reduced pressure for three hours at room temperature. Hereby N-(6-isocyanatohexyl)stearyl carbamate represented by the following formula (8) was obtained:

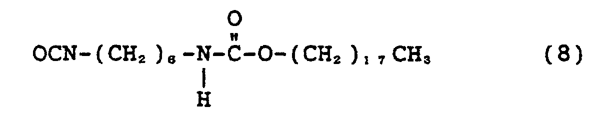

### Synthesis Example 2

### « Synthesis of pullulan-cholesterol derivative (CHP) »

A hydrophobic group-containing polysaccharide was synthesized according to the method of Akiyoshi et al {Macromolecules,. 3062 (1993)}. Thus, an eggplant type flask of 1 liter capacity was charged with 40 g (248 mmol as anhydrous glucose unit) of a pullulan (a product of Wako Pure Chemical Industries, Ltd.; average molecular weight: 108,000) and 420 ml of dimethyl sulfoxide (sometimes abbreviated as DMSO) and the mixture was agitated at 80 °C under a nitrogen atmosphere to dissolve it. To this solution, a solution of 1.78 g (3.21 mmol) of N-(6-isocyanatohexyl)cholesteryl carbamate synthesized in Synthesis Example 1-1 dissolved in 32.4 ml (0.40 mol) of pyridine was added and the mixture was subjected to reaction at 90 °C for 1.5 hours.

After termiantion of the reaction, dimethyl sulfoxide was removed by reducing the pressure and the resulting oily residue was dropped into 6 liters of acetone to form a precipitate which was purified. After removal of the supernatant, 4 liters of acetone were added to the resulting precipitate and the mixture was stood still overnight at room temperature. The precipitate was collected by filtration and was dried under a reduced pressure. The so-obtained solids were dissolved in dimethyl sulfoxide and the solution was charged in a dialysis bag (Spectra/Por3, a product of the frim Spectropor; a fractionating molecular weight of 3,500) and was subjected to a dialysis against distilled water for one week. 1.5 liters of the resulting polymer solution were treated by freeze-drying in an ordinary manner, whereby a pullulan-cholesterol derivative (abbreviated hereinafter sometimes as CHP) represented by the following formula (7a) was obtained. By calculating the proportion of introduction of the cholesteryl groups into the pullulan in the CHP from the integration value of ¹H-NMR spectrogram of CHP, it was determined that the proportion of substitution with cholesteryl group in the pullulan-cholesterol derivative (CHP) represented by the formula (7a) was about 1.3 groups per 100 monosaccharide units.

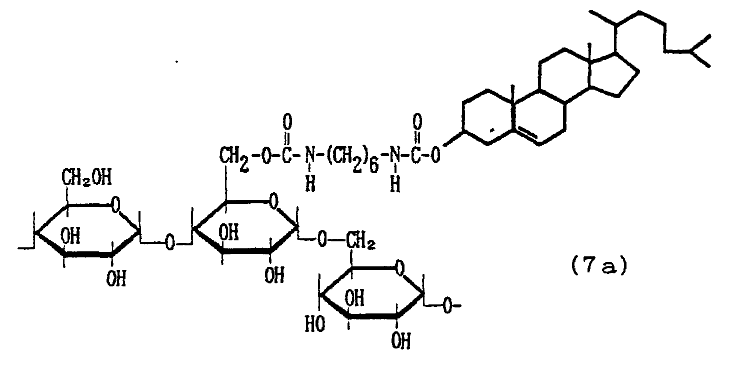

### Synthesis Example 3

By the procedures similar to those in Synthesis Example 2, a pullulan-cholesterol derivative (CHP), in which about 2.8 cholesteryl groups are introduced per 100 monosaccharide units, was synthesized.

### Synthesis Example 4

In the same manner as in Synthesis Example 2, except that a commercial mannan (a produact of the firm Sigma) having an average molecular weight of about 85,000 was used in the place of the pullulan, a mannan-cholesterol derivative (in the following, sometimes abbreviated as CHM), in which about 2.3 cholesteryl groups are introduced per 100 monosaccharide units, represented by the following formula (7b) was synthesized.

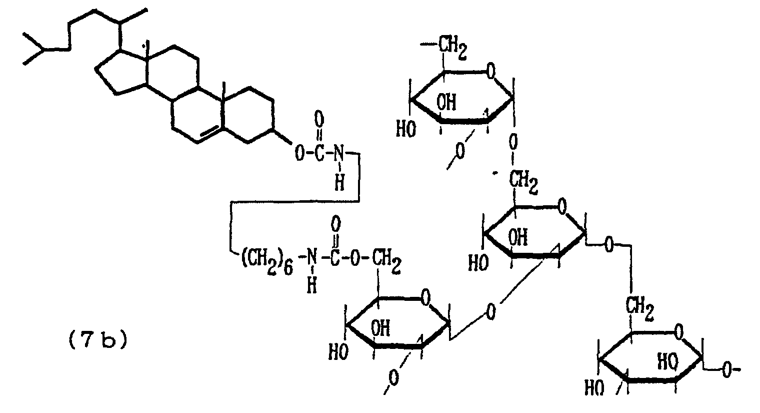

### Synthesis Example 5

In the same manner as in Synthesis Example 2, except that N-(6-isocyanatohexyl)stearyl carbamate synthesized in Synthesis Example 1-2 was used in the place of N-(6-isocyanatohexyl)cholesteryl carbamate synthesized in Synthesis Example 1-1, a stearylpullulan (in the following, sometimes abbreviated as STP), in which about 0.8 stearyl group was introduced per 100 monosaccharide units, represented by the following formula (9) was synthesized.

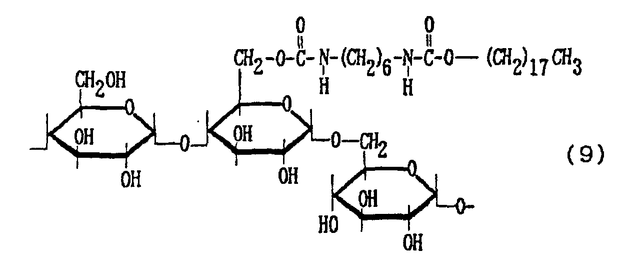

### Example 1-1

There were added 1,000 ml of water to 2 grams of the CHP obtained in Synthesis Example 2 to cause the CHP to swell at a temperature of 60 °C for 2 hours (CHP concentration = 0.2 % by weight). The resulting swollen dispersion was then stirred using a homomixer (5,000 r.p.m.) for 5 minutes. The appearance of the dispersion at this occasion was white turbid. The so-stirred swollen dispersion of 20 °C was subjected to a homogenization by causing the dispersion to spout out of an orifice under a pressure of 98 MPa (1,000 kgf/cm²) using MICROFLUIDIZER (trademark, a high pressure homogenizer Model M-110Y of the firm Mizuho Kogyo K.K.) into a chamber in order to disperse it. This homogenization treatment was repeated twice. The herein used MICROFLUIDIZER had a treating capacity of about 500 ml/min. and the time required for the twice repeats of the homogenization treatment was about 5 minutes. The resulting treated liquor had a colorless and transparent appearance. For this aqueous solution, the particle size and the number of associations were determined by the methods indicated above. The results are summarized in Tables 1 and 2.

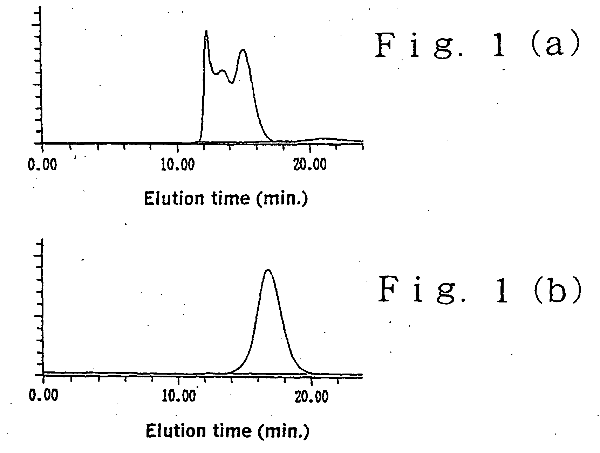

The above aqueous solution was analyzed also by a size-exclusion chromatography (SEC). The results obtained for the solution before the treatment by the high pressure homogenizer are shown in Fig. 1(a) and those after the treatment are shown in Fig. 1(b). From the results as given in Figs. 1(a) and 1(b), it was confirmed that aggregates of the CHP were formed by treating the swollen dispersion by the high pressure homogenizer.

Then, the resulting aqueous solution of the CHP aggregates was subjected to a freeze-drying, whereby the aggregates of the CHP were isolated as a white solid matter. To this solid matter, water was added so that a concentration of 0.2 % by weight would be reached, whereupon the mixture was stood still at room temperature for three hours in order to restore an aqueous solution. The restored solution was colorless and transparent. For the aqueous solution of the CHP aggregates before the freeze-drying and for the restored solution, SEC analyses were carried out, whereby it was recognized that there was no distinction in the chart curve between both the solutions and was confirmed that both are identical.

### Examples 1-2 to 1-6

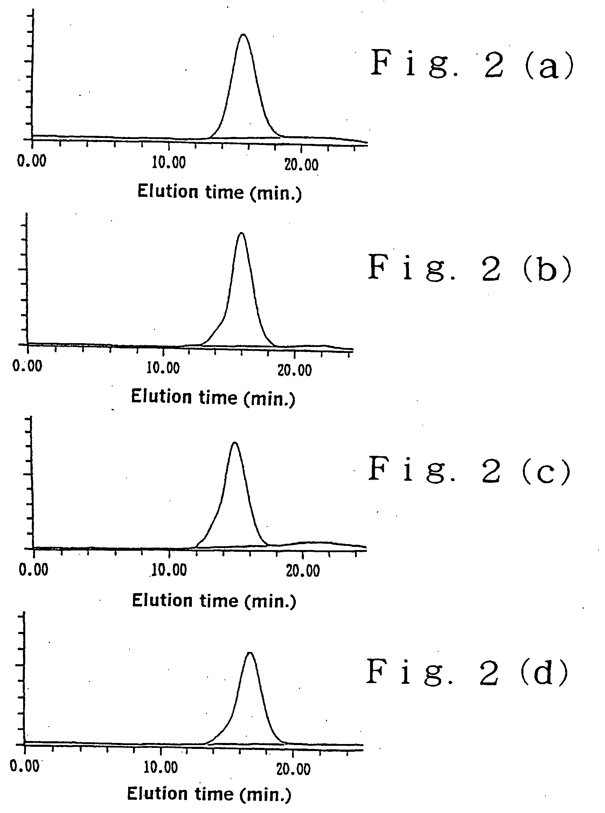

By the same procedures as in Example 1-1, homogenization treatments were carried out using the hydrophobic group-containing polysaccharides and under the conditions as recited in Table 1. The results are summarized in Tables 1 and 2. The results of SEC analysis are shown in Figs. 2(a) to 2(d). From the results as shown in Figs. 2(a) to 2(d), it was confirmed that all the Examples showed formation of the aggregate of the hydrophobic group-containing polysaccharide.

By performing the freeze-drying in the same manner as in Example 1-1, the aggregates in each Example were isolated in a form of white solid matter. For the aqueous solution of the aggregates before and after the freeze-drying, comparison was carried out as in Example 1-1, whereby it was recognized that there was no distinction therebetween and was confirmed that both are identical.

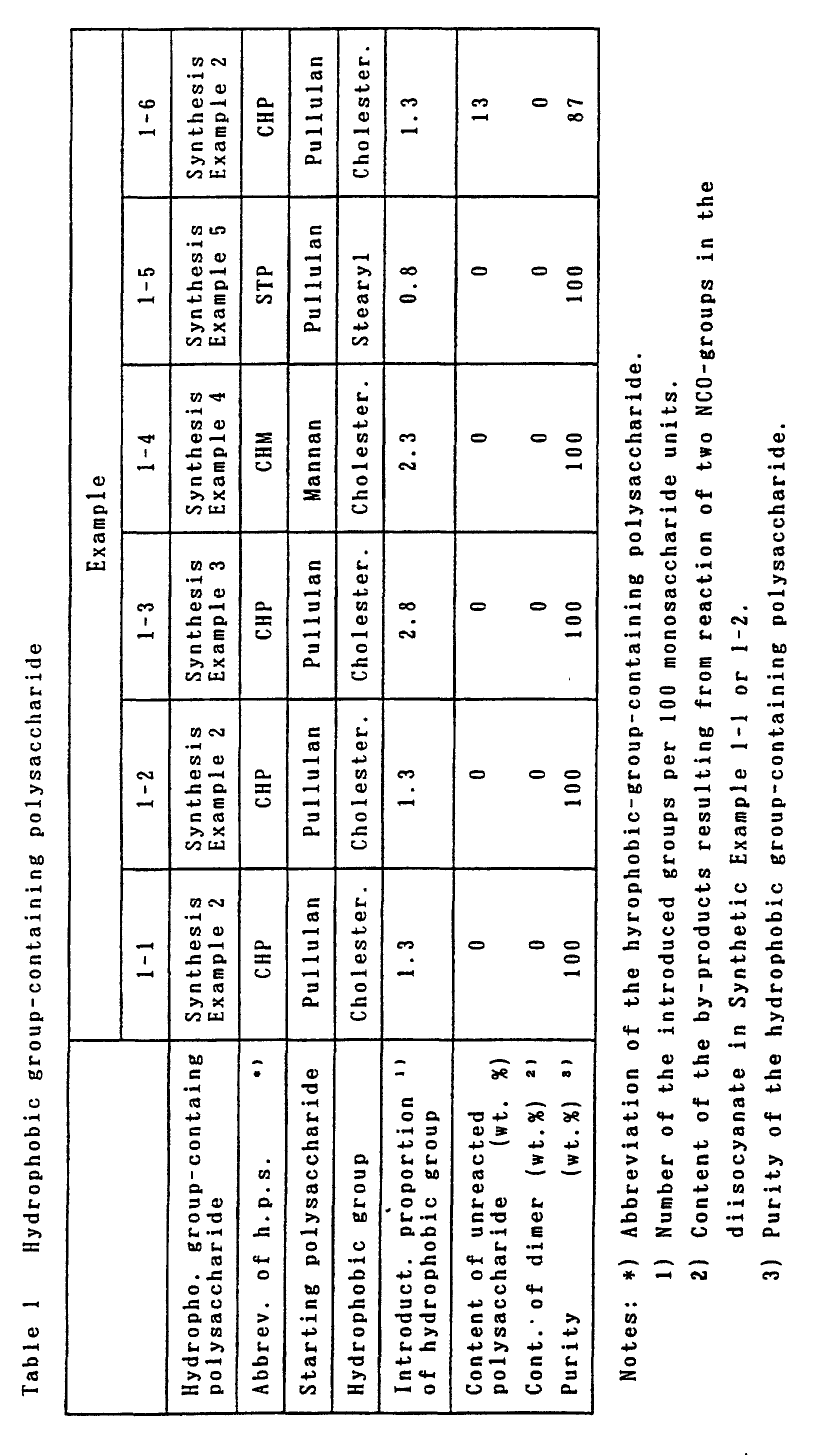

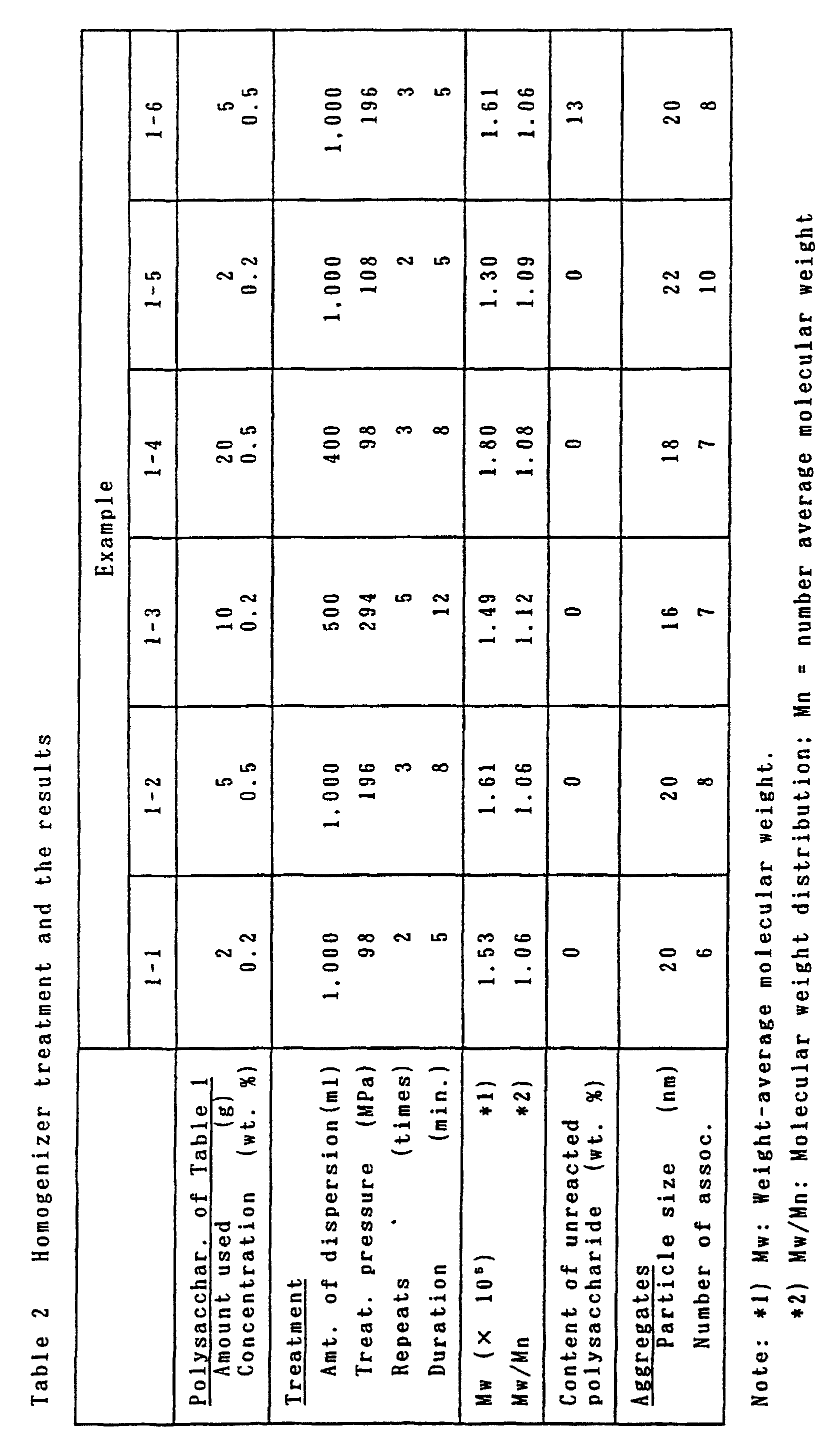

### Comparative Example 1

### « Formation of Aggregate by Dialysis»

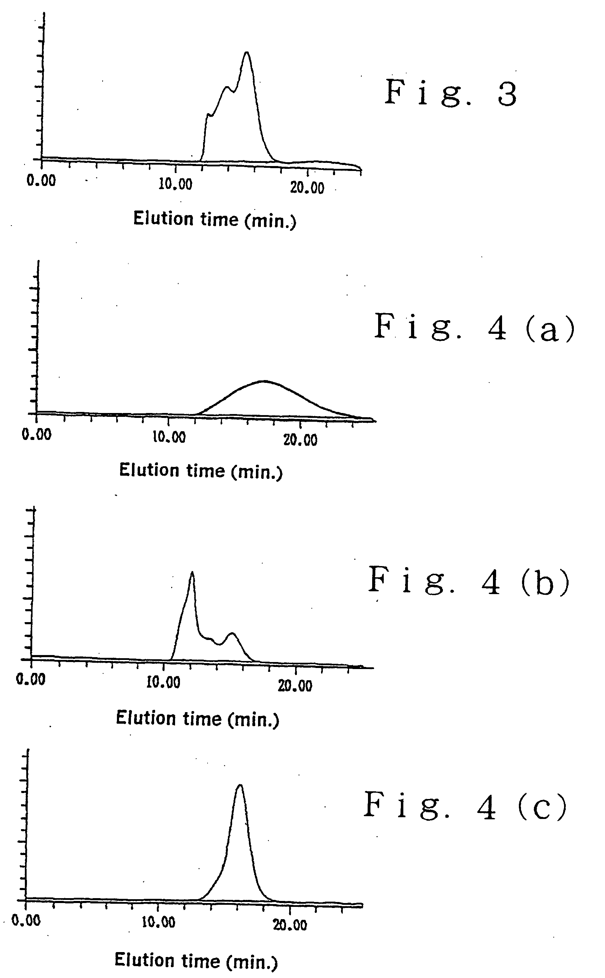

Two grams of the CHP obtained in Synthesis Example 2 were dissolved in 100 ml of dimethyl sulfoxied (DMSO). The resulting solution was charged in a dialysing bag (Spectra/Por3, supplied from the firm Spectrum; fractionating molecular weight: 3500) and was dialysed against distilled water for four days. The results of SEC analysis of the resulting dialysed liquor are shown in Fig. 3. From the results shown in Fig. 3, it is seen that monodisperse aggregates were not obtained.

As examples of aggregates of the hydrophobic group-containing polysaccharide, results of SEC analyses are shown in Figs. 4(a), 4(b) and 4(c), which were performed (a) for a pullulan having a molecular weight of 108,000; (b) for a water-dispersion of a pullulan-cholesterol derivative (CHP) based on the above pullulan (1.3 cholesteryl groups are introduced per 100 monosaccharide units of the pullulan); and (c) for the above CHP after ultrasonic wave treament after having been dispersed in water, respectively.

An elution peak is recognized for the CHP on the side of higher molecular weight than that of the pullulan, indicating occurrence of an intermolecular association. It is also seen from Figs. 4(b) and 4(c) that the CHP which was in a relatively loose association state in the dispersion was brought into formation of relatively monodisperse aggregates by the ultrasonic wave treatment. Calculation of the apparent degree of dispersion of the aggregates shown in Fig. 4(c) by reference to a standard sample of pullulan gave an Mw/Mn value of 1.1. By performing the determination by the above light-scattering for these aggregates, it has been detected that they are .microparticles having particle sizes of 15 - 25 nm in which about 8 molecules are in association.

### Comparative Example 2

### « Formation of Aggregate by Ultrasonication »

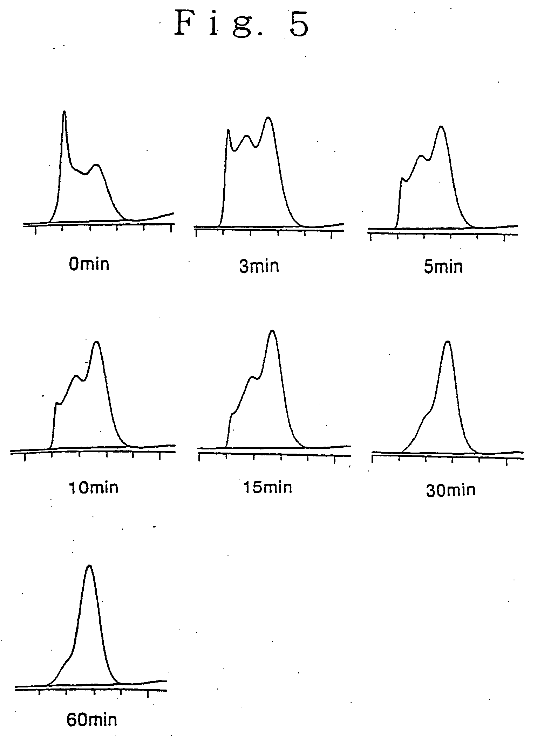

In the same manner as in Example 1-1, two grams of the CHP obtained in Synthetic Example 2 were subjected to swelling at 60 °C for two hours by introducing thereinto 1,000 ml of water (CHP concentration = 0.2 % by weight). This swollen dispersion was treated by ultrasonication using a probe-type sonicator (made of the firm Nippon Seiki; external diameter of the probe = 12 mm) at 150 W for 60 minutes. During the ultrasonication, the treating vessel was cooled from outside with ice-water to maintain the dispersion temperature always at 4 °C or lower. At each predetermined point of time (0, 3, 5, 10, 15, 30 and 60 minute) a sample of the dispersion was taken, for which SEC analyses were carried out for observing temporal variation in the formation of the aggregate. The results of SEC analyses at each occasion are shown in Fig. 5.

From the results shown in Fig. 5, it is seen that formation of aggregate was effected as the time elapsed. In the elution curve, a shoulder is seen even after 60 minutes, whereby it can be confirmed that monodisperse aggregates were not formed. When the ultrasonication was extended for further 60 minutes, the shoulder of the elution peak was recognized and no variation was seen. By analyzing this sample by the above dynamic light-scattering, it was observed that the particle size was about 128 nm.

### Reference Example 1

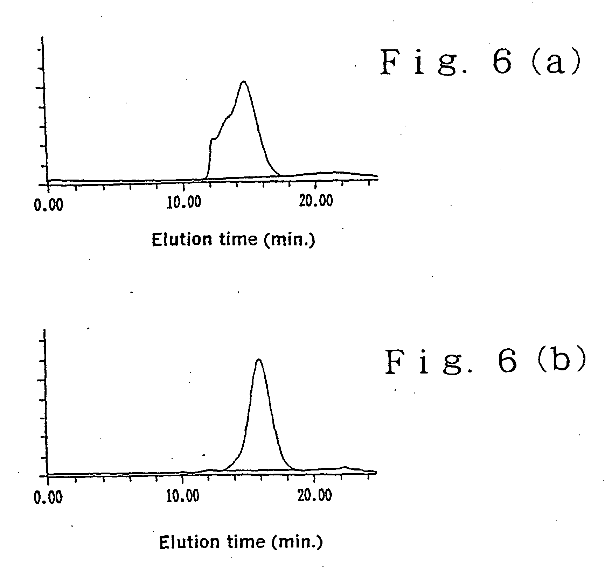

A swollen dispersion of a concentration of 0.5 % by weight of the CHP obtained in Synthesis Example 2 was prepared, which was analyzed by SEC after having been subjected to an ultrasonication under the same condition as in Comparative Example 2 for 60 minutes {Fig. 6(a)}. Here, it was shown that the form of the peak is complicated and formation of aggregate is insufficient. Therefore, it is recognizable that the effect of ultrasonication depends on the concentration. When the ultrasonication was extended for further 60 minutes, no change in the form of the peak was recognized. This ultrasonicated dispersion which showed insufficient formation of aggregate was treated using the MICROFLUIDIZER mentioned above {98 MPa (1,000 kgf/cm²); no repeat of treatment}. The results of SEC analysis of the so-treated liquor were as shown in Fig. 6(b). By further analyses by the above dynamic light-scattering and static light-scattering, it was confirmed that the particle size was about 18 nm and the number of molecules in association were about 8.

From the results given above, it is seen that a sufficient formation of aggregate was not able to attain using an ultrasonication, whereas the process as shown in Examples using a high pressure homogenizer was able to attain formation of aggregate easily.

It is also seen that aggregates exhibiting a narrower molecular weight distribution were formed in Examples 1-1 to 1-6 in which a high pressure homogenizer was used, as compared with the results of comparative Example 1 in which dialysis was employed and of Comparative Example 2 in which an ultrasonic wave treatment was used. It is further seen that aggregates of a hydrophobic group-containing polysaccharide can be formed within a brief time in a simple and convenient manner in large amount by the process according to the present invention, since inventive Example 1-1 showed a productivity of 2 grams in a treating time of 5 minutes, whereas Comparative Example 1 using dialysis showed a productivity of 2 grams in a treating time of 4 days and Comparative Example 2 using ultrasonication showed a productivity of 2 grams in a treating time of more than two hours.

The aggregates of hydrophobic group-containing polysaccharide formed by the process according to the pesent invention can be utilized as a coating material for coating drug carriers encapsulating therein drugs. For example, it can be used as the coating material for coating drug carriers, such as liposome microcapsules, microspheres, O/W emulsions and erythrocyte ghost.

## Claims

1. A process for forming aggregates of hydrophobic group-containing polysaccharide in water, comprising
causing the hydrophobic group-containing polysaccharide to swell in water and treating the resulting swollen dispersion by dispersing it using a homogenizer under a pressure of 9.8 - 490 Mpa (100-5,000 kgf/cm²),
wherein the hydrophobic group-containing polysaccharide has -XH groups (wherein X denotes oxygen atom or a nitrogen-containing group represented by NY with Y standing for hydrogen atom or a hydrocarbon group of 1 - 10 carbon atoms), wherein 0.1 - 10 -XH groups per 100 monosaccharide units constituting the polysaccharide are replaced by one or more hydrophobic groups represented by the formula (1), namely, 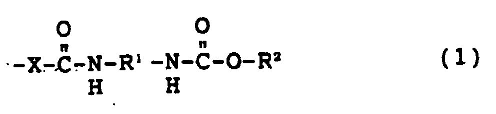 in which X is the same as given above, R¹ denotes a hydrocarbon group having 1 - 50 carbon atoms and R² denotes a hydrocarbon group having 12 - 50 carbon atoms or a steryl, and
wherein the polysaccharide to be substituted by hydrophobic group(s) consists of any one selected from pullulan, amylopectin, amylose, dextran, hydroxyethyl cellulose, hydroxyethyl dextran, mannan, levan, inulin, chitin, chitosan, xyloglucan and water-soluble cellulose.

2. A process as claimed in claim 1, wherein the homogenizer is a high-pressure homogenizer.

3. The process as claimed in claim 1, wherein the homogenizer is a high-pressure homogenizer which operates so as to jet the swollen dispersion pressurized under a pressure of 9.8 - 490 MPa (100 - 5,000 kgf/cm²) into a chamber from an orifice to disperse the swollen dispersion to treat it.

4. The process as claimed in any one of claims 1 to 3, wherein the aggregates of the hydrophobic group-containing polysaccharide have particle sizes of 10 - 30 nm and numbers of associations of the hydrophobic group-containing polysaccharide molecules of 3 - 20.

## Patentansprüche

1. Ein Verfahren zur Bildung von Aggregaten aus hydrophobe Gruppen enthaltenden Polysacchariden in Wasser, umfassend
Quellen-Lassen des hydrophobe Gruppen enthaltenden Polysaccharids in Wasser und Behandeln der resultierenden gequollenen Dispersion durch deren Dispergieren unter Verwendung eines Homogenisators unter einem Druck von 9,8 - 490 MPa (100 - 5000 kgf/cm²),
wobei das hydrophobe Gruppen enthaltende Polysaccharid -XH Gruppen aufweist (wobei X ein Sauerstoffatom oder eine Stickstoff enthaltende Gruppe, die durch NY dargestellt wird, wobei Y für ein Wasserstoffatom oder eine Kohlenwasserstoffgruppe von 1 - 10 Kohlenstoffatomen steht, bezeichnet), wobei 0,1 - 10 -XH Gruppen pro 100 Monosaccharideinheiten, welche das Polysaccharid bilden, ersetzt sind durch eine oder mehrere hydrophobe Gruppen, die durch die folgende Formel (1) dargestellt sind, nämlich 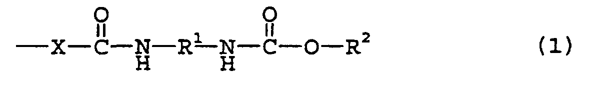 worin X genau so wie oben dargelegt ist, R¹ eine Kohlenwasserstoffgruppe mit 1 - 50 Kohlenstoffatomen bezeichnet und R² eine Kohlenwasserstoffgruppe mit 12 - 50 Kohlenstoffatomen oder ein Steryl bezeichnet, und
wobei das mittels hydrophober Gruppe(n) zu substituierende Polysaccharid aus irgendeinem besteht, das ausgewählt ist aus Pullulan, Amylopektin, Amylose, Dextran, Hydroxyethylcellulose, Hydroxyethyldextran, Mannan, Levan, Inulin, Chitin, Chitosan, Xyloglucan und wasserlöslicher Cellulose.

2. Ein Verfahren nach Anspruch 1, wobei der Homogenisator ein Hochdruckhomogenisator ist.

3. Das Verfahren nach Anspruch 1, wobei der Homogenisator ein Hochdruckhomogenisator ist, der so funktioniert, dass die gequollene Dispersion, die mit einem Druck von 9,8 - 490 MPa (100 - 5000 kgf/cm²) unter Druck steht, aus einer Düse in eine Kammer gestrahlt wird, um die gequollene Dispersion zu dispergieren, um sie zu behandeln.

4. Das Verfahren nach einem der Ansprüche 1 bis 3, wobei die Aggregate des hydrophobe Gruppen enthaltenden Polysaccharids eine Teilchengröße von 10 - 30 nm und Assoziationszahlen der hydrophobe Gruppen enthaltenden Polysaccharidmoleküle von 3 - 20 aufweisen.

## Revendications

1. Procédé pour former des agrégats de polysaccharide contenant des groupes hydrophobes dans l'eau, comprenant
le gonflement du polysaccharide contenant des groupes hydrophobes dans l'eau et le traitement de la dispersion gonflée résultante par dispersion de celle-ci au moyen d'un homogénéisateur sous une pression de 9,8 - 490 Mpa (100 - 5000 kgf/cm²),
où le polysaccharide contenant des groupes hydrophobes a des groupes -XH (où X désigne un atome d'oxygène ou un groupe contenant de l'azote représenté par NY où Y représente un atome d'hydrogène ou un groupe hydrocarboné de 1 - 10 atomes de carbone), où 0,1 - 10 groupes -XH pour 100 unités de monosaccharide constituant le polysaccharide sont remplacés par un ou plusieurs groupes hydrophobes représentés par la formule (1), à savoir 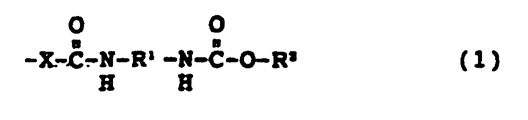 où X est le même qu'indiqué ci-dessus, R¹ désigne un groupe hydrocarboné ayant 1 - 50 atomes de carbone et R² désigne un groupe hydrocarboné ayant 12 - 50 atomes de carbone ou un stéryle, et
où le polysaccharide destiné à être substitué par un ou des groupes hydrophobes consiste en un polysaccharide quelconque choisi parmi le pullulane, l'amylopectine, l'amylose, le dextrane, l'hydroxyéthylcellulose, l'hydroxyéthyldextrane, le mannane, le lévane, l'inuline, la chitine, le chitosane, le xyloglucane et la cellulose hydrosoluble.

2. Procédé selon la revendication 1 où l'homogénéisateur est un homogénéisateur haute pression.

3. Procédé selon la revendication 1 où l'homogénéisateur est un homogénéisateur haute pression qui opère de manière à projeter la dispersion gonflée sous pression sous une pression de 9,8 - 490 MPa (100 - 5000 kgf/cm²) dans une chambre depuis un orifice pour disperser la dispersion gonflée pour la traiter.

4. Procédé selon l'une quelconque des revendications 1 à 3 où les agrégats du polysaccharide contenant des groupes hydrophobes ont des tailles de particules de 10 - 30 nm et des nombres d'associations des molécules de polysaccharide contenant des groupes hydrophobes de 3 - 20.
